# FASCICULE DE BREVET EUROPEEN

(11) **EP 2 254 506 B1**
(45) Date de publication et mention de la délivrance du brevet: **23.12.2015**
(21) Numéro de dépôt: 09720642.9
(22) Date de dépôt: 25.02.2009
(51) Int. Cl.: A61F 2/06, A61B 17/11

(54) **DISPOSITIF PROTHETIQUE POUR REALISER UN PONTAGE VASCULAIRE, SANS CLAMPAGE, A PARTIR D'UN VAISSEAU SANGUIN**
PROTHESE ZUR HERSTELLUNG EINS GEFÄSSBYPASSES AUS EINEM BLUTGEFÄSS OHNE KLEMMEN
PROSTHETIC DEVICE FOR CREATING A VASCULAR BYPASS, WITHOUT CLAMPING, FROM A BLOOD VESSEL

(30) Priorité: 28.02.2008 FR 0801104
(43) Date de publication de la demande: 01.12.2010
(73) Titulaire: Vascular Office (SARL) Technopole de Chateau Gombert, 13382 Marseille Cedex 13 (FR)
(72) Inventeur: FORMICHI, Maxime, F-13007 Marseille (FR)
(74) Mandataire: Marek, Pierre
(86) Numéro de dépôt international: PCT/FR2009/000201
(87) Numéro de publication internationale: WO 2009/112721

(56) Documents cités:
- EP-A- 0 269 254
- US-B1- 6 503 258

## Description

La présente invention concerne un dispositif prothétique pour réaliser un pontage vasculaire, sans clampage, à partir d'un vaisseau sanguin en particulier à partir de l'aorte et pouvant présenter des branches collatérales importantes. Plus particulièrement, l'invention vise une prothèse pour revasculariser les branches collatérales de l'aorte, sans clampage, c'est-à-dire sans interruption de la circulation sanguine dans celle-ci, au moyen de pinces occlusives ou clamps, ou autres.

Dans cette application, lorsqu'on veut apporter du sang dans une artère qui naît de l'aorte, parce que celle-ci est occluse plus ou moins complètement, il existe plusieurs procédés qui sont utilisés plus ou moins préférentiellement suivant les circonstances pathologiques.

Quand l'artère présente un rétrécissement ou une occlusion isolée et courte, une méthode utilisant une dilatation par un ballonnet associé à la mise en place d'une endoprothèse, est préconisée. Cette endoprothèse (encore appelée stent) est une sorte de tube grillagé destiné à maintenir perméable la zone qui a été dilatée et qui était le siège de la lésion. Cette méthode est non invasive. Elle se fait à l'aide de cathéters introduits dans les artères, le plus souvent sous anesthésie locale. Il n'y a pas d'ouverture chirurgicale. Le saignement est pratiquement inexistant.

Souvent la maladie est plus développée, atteignant une branche de l'aorte sur une grande longueur, ou plusieurs branches, voire même intéresse la portion d'aorte d'où naissent les branches. Il peut alors être nécessaire d'apporter le sang aux organes irrigués par cette ou ces artères en réalisant un ou des pontages vasculaires à partir de l'aorte. Ces pontages (ou by-passes) sont réalisés, en général, par des conduits prothétiques. Ils permettent de court-circuiter les lésions. Une des extrémités du conduit prothétique dite proximale est cousue sur l'aorte. C'est elle qui capte le sang. L'autre extrémité dite "distale" est cousue sur la branche de l'aorte en aval des lésions. C'est elle qui distribue le sang. Ces zones de coutures sont appelées anastomoses.

L'anastomose proximale sur l'aorte est réalisée en cousant le tube prothétique sur un orifice découpé dans la paroi aortique. Cet orifice, encore appelé spécifiquement aortotomie lorsqu'il s'agit de l'aorte, doit être isolé de la circulation pendant tout le temps de la couture pour éviter le saignement. Pour cela, on arrête la circulation dans l'aorte à l'aide de pinces spéciales appelées clamps. On réalise alors, soit un clampage qui peut être total : dans ce cas, la circulation est complètement stoppée, ou alors un clampage dit partiel laissant passer un peu de sang pour les territoires situés en aval. De même, l'anastomose distale sur la branche collatérale se fait en isolant de la circulation, le segment d'artère siège de l'anastomose, et en pratiquant une ouverture ou section de celle-ci.

Ces procédés de pontages existent depuis plusieurs dizaines d'années.

Ils nécessitent :
1/ Une incision chirurgicale de la paroi abdominale et/ou de la paroi thoracique, selon les cas, pour accéder à l'aorte et parfois une autre incision (ou plusieurs) pour accéder à la branche (ou aux branches) que l'on veut revasculariser.
2/ D'arrêter partiellement ou complètement la circulation dans l'aorte pour réaliser la suture ou anastomose du tube prothétique à ce niveau ; tout arrêt complet ou partiel de la circulation dans l'aorte ayant des répercussions hémodynamiques et métaboliques importantes sur tous les organes situés en aval et en amont.

En aval : les organes vont être privés plus ou moins complètement de sang, et donc d'oxygène, pendant toute la durée de l'anastomose. Cet arrêt de la circulation est plus ou moins délétère suivant l'endroit où est pratiqué l'arrêt de la circulation.

À titre d'exemple, un arrêt de la circulation au niveau de la partie terminale de l'aorte située dans l'abdomen est bien toléré pendant quelques heures. En aval, il ne reste plus que les membres inférieurs et les organes du petit bassin peu sensibles à ce que l'on appelle une absence de circulation ou ischémie (privation d'oxygène).

Par contre, plus l'arrêt de la circulation se situe plus haut (en amont) sur l'aorte, plus la tolérance est limitée.

Ainsi si on arrête la circulation au niveau de la partie basse du thorax, on prive aussi de sang artériel, une partie de la moelle épinière, ainsi que tous les organes situés dans l'abdomen tels que, par exemple, les reins, le foie, le tube digestif. Ces organes ne supportent l'ischémie que pendant quelques dizaines de minutes au maximum. Le temps pour réaliser l'anastomose est donc très limité.

Le maximum de conséquences néfastes se manifeste lorsque l'on arrête la circulation au niveau de l'origine de l'aorte, juste après sa naissance. En plus de priver les autres territoires déjà cités précédemment situés en aval, cette portion de l'aorte donne naissance aux artères qui irriguent le cerveau. Ces artères ne tolèrent que deux à trois minutes d'arrêt circulatoire, ce qui rend impossible la réalisation de l'anastomose. Ainsi l'arrêt circulatoire n'est possible à ce niveau qu'en faisant appel à des techniques d'assistance circulatoire extracorporelle utilisant des machines. Ces moyens mis en oeuvre pendant la suture, continuent d'apporter du sang au niveau du cerveau ainsi que dans les territoires déjà mentionnés pour éliminer les effets délétères de l'arrêt de la circulation dans l'aorte pour réaliser l'implantation du pontage prothétique. Malheureusement, ces techniques d'assistance circulatoire sont lourdes. Elles sont elles-mêmes la cause de complications propres et d'une mortalité importante.

En amont, l'interruption du flux artériel a pour conséquence de créer une hyperpression qui se manifeste dans le territoire situé au-dessus. Comme pour l'ischémie, l'hyperpression est d'autant plus importante que l'on travaille plus haut. Elle entraîne une surcharge de travail du coeur.
3/ De suturer le tube prothétique et la paroi aortique bord à bord avec un fil très fin. Lorsqu'on remet en circulation la zone de fixation, il y a un risque d'hémorragie. En effet il est rare qu'une suture soit étanche d'emblée et il faut souvent interrompre de nouveau la circulation et rajouter des points sur la suture. Cet élément est à prendre en considération de manière importante de nos jours en raison de la fréquence des traitements antiagrégants et anticoagulants qui sont pratiquement systématiques et prescrits à vie chez les patients atteints de maladies cardiovasculaires. Ces traitements qui favorisent le saignement, ne peuvent pas toujours être arrêtés pendant l'opération.

On remarque, d'autre part, que ce type de suture bord à bord présente d'autres inconvénients en particulier à long terme. La suture est soumise en permanence à la pression artérielle avec 70 fois par minute une augmentation momentanée due aux contractions du coeur. Il apparaît parfois des déchirures, soit de la paroi aortique, soit de la prothèse avec une déhiscence de la jonction artère prothèse. Cette déhiscence, une fois amorcée, va se développer toujours sous l'effet de la pression artérielle. Elle conduit, dans environ 2% des cas, à la formation d'un faux anévrysme qui nécessite le plus souvent une nouvelle opération.
4/ De suturer l'extrémité distale du tube prothétique sur la ou les branches collatérales auxquelles est destiné le sang. Cette partie de l'opération est un geste moins traumatisant par rapport à la suture aortique.

On a proposé des dispositifs prothétiques pour faciliter l'anastomose et limiter certains de ses risques (FR-2.799.362, EP-0.269.254, US-6.273 912, WO-2006/013234, FR-2.751.867). Ces dispositifs comprennent un tube prothétique généralement réalisé en textile synthétique compatible avec les tissus biologiques et, pour cela, dit "biocompatibles", comportant ou non une ou plusieurs ramifications et muni, à ses extrémités, d'une collerette adaptée pour l'anastomose dudit tube prothétique sur un conduit organique tel qu'une artère (aorte ou autre) et/ou une veine. Ces collerettes permettent la fixation de la prothèse sur un ou deux conduits organiques, par une suture classique ou à l'aide de clips ou d'agrafes, en regard d'ouvertures (artériotomies) pratiquées dans le ou lesdits conduits organiques. Dans tous les cas, la face interne de ces collerettes est en contact avec l'intérieur du vaisseau sanguin et le sang circulant.

Toutefois, la procédure opératoire utilisant ces dispositifs prothétiques nécessite toujours un clampage ou interruption de la circulation sanguine dans la portion du conduit anatomique ou l'on veut implanter la prothèse, avec les conséquences graves que cela peut entraîner, tandis que les risques de saignements per et postopératoires restent importants.

Dans le document US 6.503.258, est décrit un dispositif pour anastomose vasculaire, en particulier pour implanter une veine sur une artère (par exemple : aorte).

Ce dispositif comprend ; - une collerette disposée à la base d'un élément tubulaire, dans un plan généralement perpendiculaire à ce dernier, ladite collerette comportant une pluralité d'ouvertures disposées autour de sa partie centrale, de sorte à permettre sa fixation par suture sur une artère, ledit dispositif permettant à un greffon veineux d'être inséré et maintenu au sein de l'organe tubulaire non suturé, lequel est de diamètre variable pour accueillir des greffons veineux de diamètres variables.

La mise en oeuvre de ce dispositif nécessite l'introduction de l'extrémité du greffon veineux dans l'aorte, à travers une incision préalablement pratiquée dans celle-ci, ce qui conduit à la nécessité d'interrompre la circulation sanguine dans le vaisseau artériel, au moyen d'un clampage, pendant la mise en place du dispositif et durant l'exécution de la suture, avec les risques importants qui peuvent se concrétiser si cette interruption dépasse la durée tolérable. D'autre part, la fixation de la collerette sur l'artère ne présente aucune garantie d'étanchéité et peut donc entraîner des hémorragies, ce qui n'est pas concevable dans le domaine de la chirurgie.

Le dispositif décrit dans le document US 6.503.258 est donc totalement inapplicable pour la réalisation de pontages à partir de l'aorte.

Un objet de la présente invention est constitué par un dispositif prothétique dont la fixation sur une artère, en particulier sur l'aorte, peut s'opérer sans clampage arrêtant la circulation et sans risque de saignements per et postopératoires.

Selon une disposition caractéristique, le premier objectif susmentionné est atteint grâce à un dispositif prothétique comprenant un ou plusieurs tubes prothétiques dont l'une des extrémités est solidaire d'une collerette de fixation souple comportant une face externe et une face interne destinée à être appliquée sur la paroi externe dudit vaisseau artériel, cette collerette étant pourvue d'un ou plusieurs orifices disposés dans une zone centrale dite zone des orifices lesquels communiquent avec le ou lesdits tubes prothétiques, cette collerette comportant une zone de solidarisation disposée autour de ladite zone des orifices, à distance du bord dudit ou desdits orifices et étant destinée à être fixée sur la paroi externe d'un vaisseau artériel, la face interne de cette zone de solidarisation étant pourvue d'au moins un moyen permettant de créer une zone annulaire d'étanchéité autour de ladite zone des orifices.

Selon un premier mode d'exécution, la face interne de la zone de solidarisation présente au moins un joint souple annulaire disposé autour de la zone des orifices.

Selon un deuxième mode d'exécution, la zone de solidarisation comporte, sur sa face interne, un espace ou loge annulaire disposée autour de là zone des orifices et permettant l'injection d'un produit, par exemple d'une colle et/ou d'un produit hémostatique avantageusement bio-résorbable, destiné à constituer une zone de fixation annulaire formant joint étanche.

Selon un mode d'exécution préféré, le joint annulaire est réalisé en silicone ou en polyuréthanne.

Selon un mode d'exécution possible, la loge annulaire est délimitée latéralement par deux joints concentriques espacés.

Selon une autre disposition caractéristique, la loge annulaire est délimitée par une bande annulaire inférieure perméable destinée à être appliquée sur la paroi d'un vaisseau sanguin en particulier sur la paroi aortique, cette bande annulaire étant pourvue de petits trous permettant la diffusion, en sous-face de ladite collerette, d'une colle biologique et/ou d'un produit hémostatique injecté dans ladite loge.

Selon une autre disposition caractéristique, la loge annulaire est constituée par une bande de tissu biocompatible munie de petits trous et solidarisée par ses bords latéraux à la face inférieure de la collerette.

Selon un mode d'exécution intéressant, la bande annulaire constituant la face inférieure de la loge est exécutée dans un matériau bio-résorbable.

Selon un autre mode d'exécution, la loge annulaire est disposée à l'extérieur du joint souple.

Selon un autre mode d'exécution, la collerette comporte, en sous-face, un joint annulaire disposé à l'extérieur de la loge annulaire.

Selon un mode d'exécution intéressant, au moins un cathéter souple est disposé dans la loge annulaire pour l'injection d'une colle biologique et/ou d'un produit hémostatique, l'extrémité proximale de ce cathéter traversant un pertuis ménagé dans la collerette, et étant accessible sur la face externe de cette dernière, de sorte à permettre son raccordement à un réservoir de colle biologique et/ou de produit hémostatique et son retrait lors de l'injection des produits susmentionnés.

Suivant une disposition avantageuse, deux cathéters souples sont disposés, avec une disposition inverse, dans la loge ménagée en sous-face de la collerette de fixation, l'un pour l'injection d'une colle biologique, l'autre pour l'injection d'un produit catalysant, ces cathéters pouvant être retirés successivement en suivant des trajets inverses (dextrorsum et senestrorsum).

Selon une autre disposition caractéristique, la face interne ou face inférieure de la collerette présente un épaississement annulaire disposé autour de la zone des orifices, entre cette zone et l'emplacement de la loge annulaire.

L'invention permet notamment d'éviter l'arrêt de la circulation sanguine dans l'aorte durant l'intervention et de réduire le risque de saignement lorsque l'on effectue la revascularisation des branches collatérales de l'aorte par un pontage à partir de cette dernière. Ce résultat est dû au mode de fixation de la collerette sur la paroi de l'aorte qui s'effectue sans aucune effraction sur celle-ci

Ces solutions permettent de diminuer la lourdeur de l'opération et d'éliminer ainsi la mortalité inhérente à l'arrêt circulatoire et aux hémorragies.

Le dispositif de l'invention peut, en outre, être appliqué à la revascularisation des branches de l'aorte lorsque celles-ci naissent d'une aorte pathologique présentant ce que l'on appelle un anévrysme. Dans ces conditions, la portion d'aorte anévrysmale doit être remplacée et la circulation artérielle dans les branches collatérales doit être rétablie rapidement. Le dispositif de l'invention peut permettre de revasculariser les branches de l'aorte avec les avantages déjà décrits. Le remplacement de la partie malade de l'aorte pouvant, lui, se faire après cette revascularisation. Ce remplacement peut faire appel, selon l'anatomie de l'anévrysme, à la chirurgie ou avantageusement à la mise en place d'une endoprothèse aortique. Dans cette dernière solution qui est la moins agressive pour le patient, le procédé fait appel à une technique mini invasive par cathéter. La présence du dispositif prothétique de l'invention préalablement implanté sur l'aorte en amont ou en aval permet de faciliter en outre la mise en place de l'endoprothèse. Il est possible, en effet, d'utiliser l'un des tubes prothétiques pour introduire l'endoprothèse dans l'aorte sans avoir recours à une introduction du cathéter par la voie fémorale. C'est un avantage supplémentaire qui sera décrit plus en détail ci-après.

Un autre avantage du dispositif prothétique selon l'invention découle du fait que la collerette de ce dispositif ne se trouve à aucun moment en contact avec le sang circulant dans le vaisseau artériel sur lequel elle est fixée, ladite collerette étant uniquement en contact avec la paroi externe dudit vaisseau.

Les buts, caractéristiques et avantages ci-dessus, et d'autres encore, ressortiront mieux de la description qui suit et des dessins annexés dans lesquels :
La figure 1 est une vue en perspective d'un exemple de réalisation du dispositif prothétique selon l'invention qui, selon cet exemple, comporte trois branches collatérales ou conduits prothétiques.
La figure 2 est une vue en coupe axiale de ce dispositif.
La figure 3 est une vue de dessus et en coupe selon la ligne 3-3 de la figure 2, montrant la face externe de la collerette de fixation du dispositif.
La figure 4 est une vue de dessous, montrant la face interne de cette collerette.
Les figures 5 et 6 sont des vues analogues aux figures 3 et 4, respectivement, illustrant une variante de réalisation du dispositif prothétique.

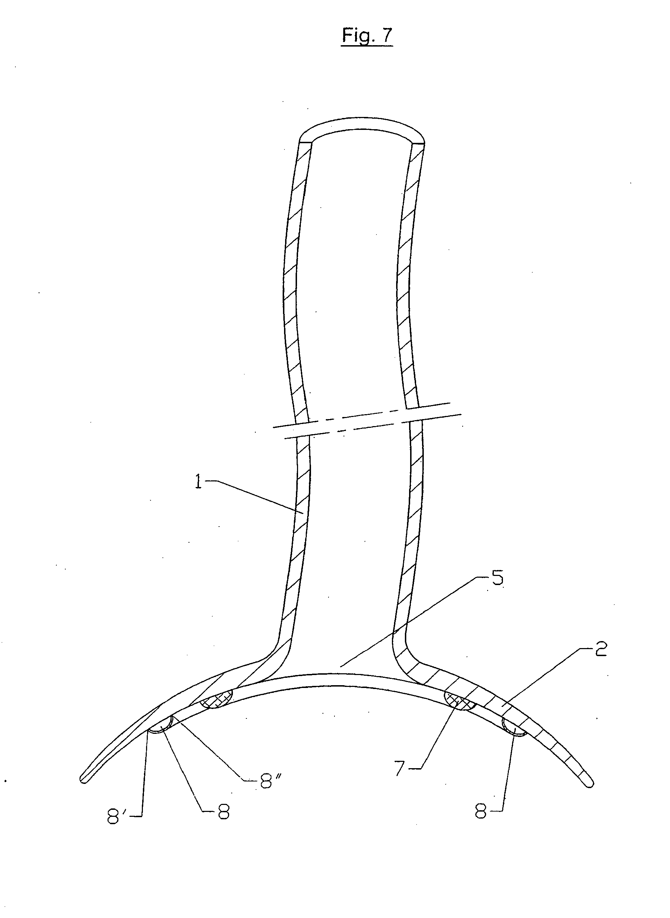

La figure 7 est une vue à plus grande échelle et en coupe selon la ligne 7-7 de la figure 3.

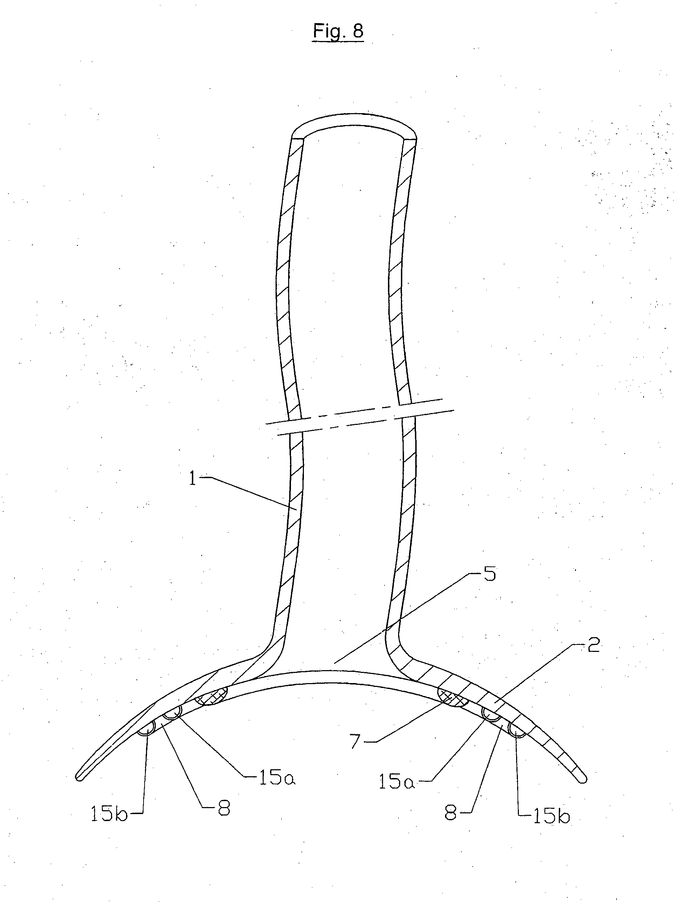

Les figures 8 et 9 sont des vues analogues à la figure 7 et illustrant deux variantes de réalisation de la surface de la collerette de fixation.
La figure 10 est une vue à caractère schématique montrant un exemple de fixation du dispositif de l'invention sur un vaisseau artériel.
La figure 11 est une vue en coupe transversale, à plus grande échelle, montrant la fixation de ce dispositif sur une aorte.
La figure 12 est une vue à caractère schématique illustrant l'installation d'un stent dans un tel dispositif prothétique.
La figure 13 est une vue partielle, en coupe, montrant un autre mode d'exécution des conduits prothétiques.

On se réfère auxdits dessins pour décrire des modes d'exécution intéressants, bien que nullement limitatifs, du dispositif prothétique selon l'invention.

On précise que si l'on décrit ci-après une application particulièrement intéressante de l'invention à des pontages réalisés à partir de l'aorte, et plus particulièrement à partir de la crosse aortique, elle est également applicable pour l'exécution de pontages à partir d'autres vaisseaux artériels.

Ce dispositif comprend au moins un tube ou conduit prothétique souple 1 a dont l'une des extrémités est solidaire d'une collerette souple 2 comportant une face externe 3 et une face interne 4. Selon les applications, le dispositif peut comporter plusieurs tubes prothétiques. Notamment dans l'application à la revascularisation des branches collatérales de l'aorte, ce dispositif possède trois tubes ou conduits prothétiques souples 1 a, 1b, 1 c rattachés à la collerette souple 2. Il peut posséder plus de trois tubes ou conduits prothétiques souples par exemple quatre tubes ou conduits.

La collerette souple 2 est pourvue, dans sa partie centrale, d'un ou plusieurs orifices 5 dont le nombre correspond à celui des conduits prothétiques 1 a, 1 b, 1c. Selon les exemples représentés, la collerette 2 est pourvue de trois orifices 5a, 5b, 5c, chacun de ceux-ci communiquant avec l'un des conduits prothétiques 1a, 1b, 1c, respectivement.

La collerette souple 2 présente une forme permettant sa mise en contact intime avec la paroi externe du vaisseau artériel sur lequel elle doit être fixée.

La collerette souple 2 présente ainsi une forme et des dimensions qui peuvent varier en fonction de la forme et de la taille des conduits anatomiques à l'emplacement où il s'avère souhaitable d'implanter le dispositif prothétique. Cette forme est approximativement celle d'une portion de cylindre droit à angles arrondis si le dispositif doit être implanté sur une portion d'aorte rectiligne ou celle d'une portion de cylindre coudé, plus ou moins courbe, lorsque ledit dispositif doit être raccordé à la portion courbe en crosse d'évêque de l'aorte appelée arche aortique.

La collerette souple 2 est réalisée en tissus prothétiques habituellement utilisés pour les prothèses vasculaires tels que, par exemple, Dacron (marque déposée) ou autre fibre polyester analogue, PTFE (par exemple Goretex : marque déposée), polyuréthanne ou tout autre matériau compatible biologiquement.

Comme indiqué précédemment, la collerette souple 2 peut être munie de plus de trois conduits prothétiques, dans ce cas, elle est également pourvue de plus de trois orifices.

Cette collerette pourvue des orifices 5a, 5b, 5c, comporte, comme indiqué précédemment, deux faces opposées, soit :
- une face externe 3, visible après implantation du dispositif et,
- une face interne 4, destinée à être placée au contact de l'aorte.

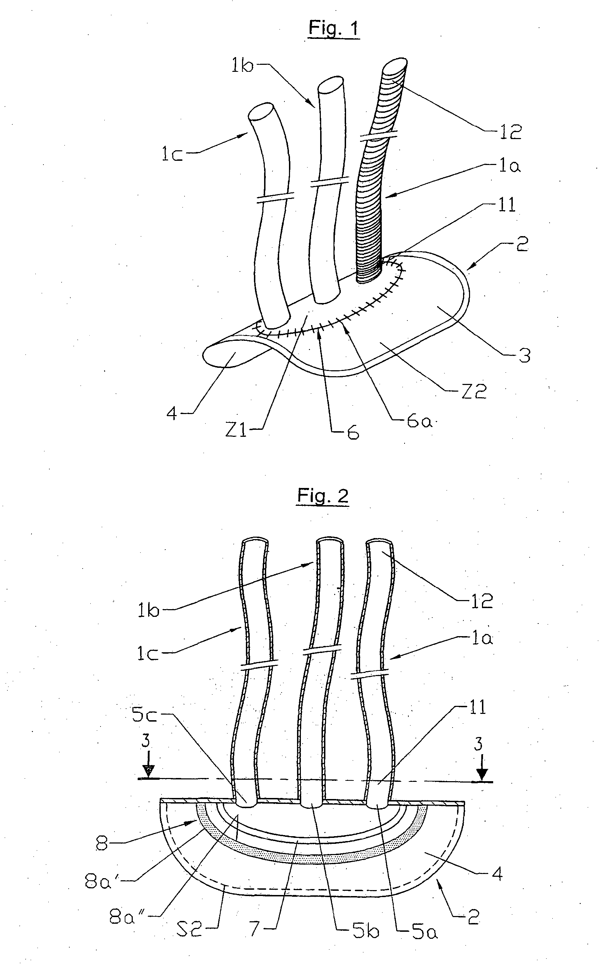

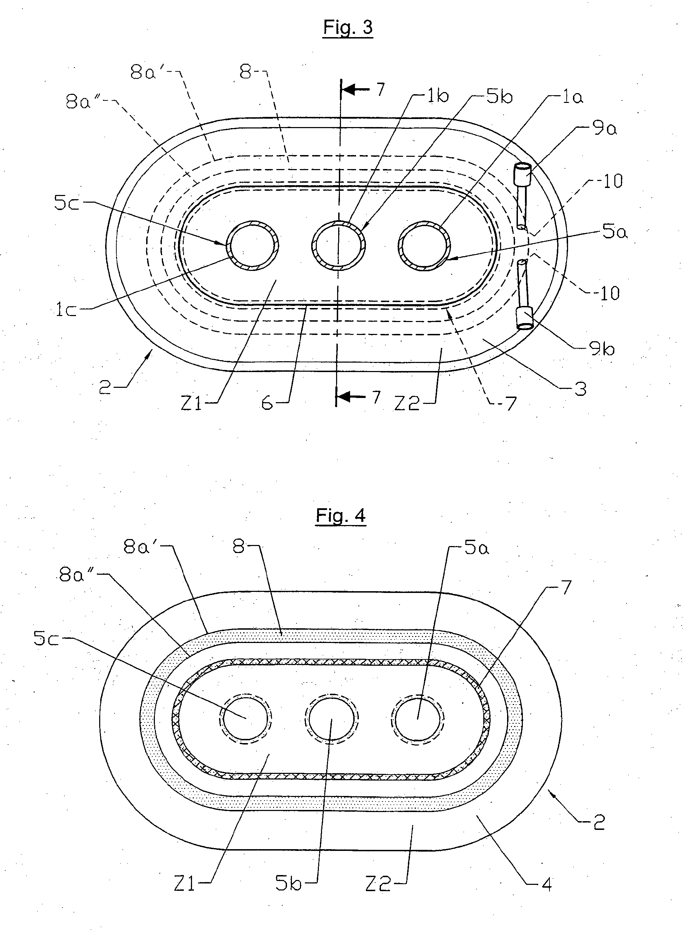

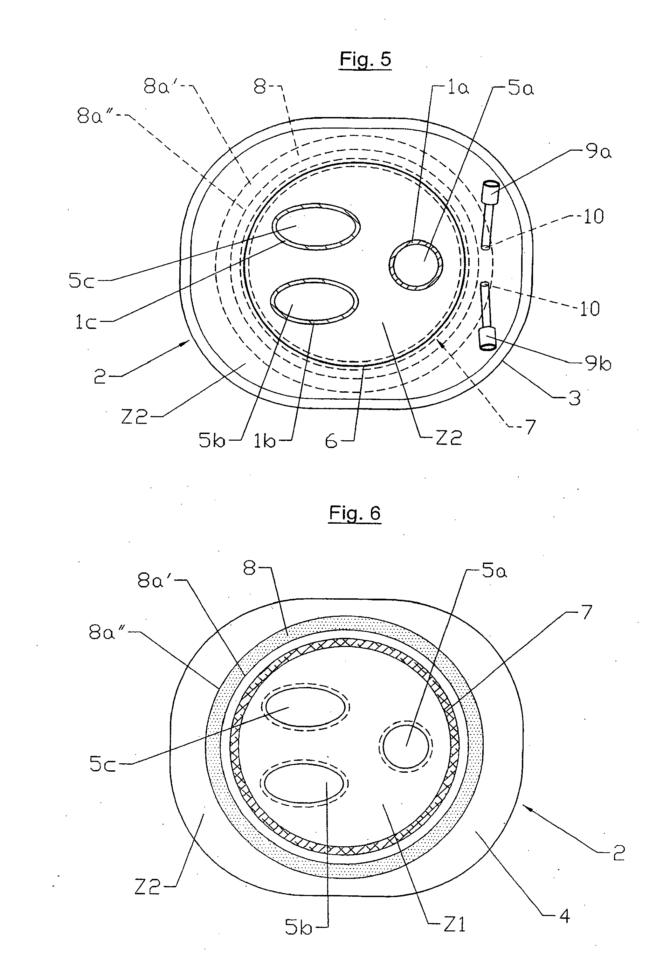

Les orifices 5a, 5b, 5c, peuvent être alignés régulièrement sur la partie médiane de la collerette (figures 1 à 4) correspondant à l'axe du vaisseau et à la circulation, ou placés différemment (figures 5 et 6). Quelle que soit leur disposition, ils occupent la zone centrale de la collerette appelée zone des orifices Z1 dans la suite de la description. La partie de la collerette située autour de la zone des orifices Z1 est destinée à être solidarisée à la paroi de l'aorte et appelée zone de solidarisation Z2, laquelle se trouve ainsi disposée à distance du bord du ou des orifices 5a, 5b, 5c.

Chaque orifice 5a, 5b, 5c, se prolonge par un tube prothétique 1a, 1b, 1c, dont le calibre ou section est équivalent à celui de l'artère à revasculariser. Le tube prothétique 1a, 1b, 1c est solidarisé sur le bord de l'orifice correspondant 5a, 5b, 5c, par couture au fil, collage, thermo soudure ou tout autre procédé approprié donnant l'étanchéité et la solidité nécessaires. L'angle d'implantation d'un tube prothétique 1 a, 1b, 1c, au niveau d'un orifice 5a, 5b, 5c, peut être compris entre 1° et 90°. La forme de l'orifice ou de chaque orifice est adaptée à l'angle d'implantation pour obtenir la meilleure hémodynamique sanguine. L'orifice pourra être en forme de cercle pour une implantation du tube prothétique proche de 90° ; il pourra être de forme ovalisée plus ou moins prononcée si l'angle d'implantation du tube prothétique sur la collerette est inférieur à 90°. Dans ce dernier cas, le grand axe des orifices ovalisés peut avoir différentes orientations en fonction de la direction des conduits prothétiques selon l'emplacement du raccordement de l'extrémité distale desdits conduits sur les branches collatérales de l'aorte.

La longueur des tubes prothétiques 1a, 1 b, 1c solidarisés sur le bord des orifices 5a, 5b, 5c sera adaptée à la distance pour rejoindre l'artère à revasculariser.

La zone des orifices Z1 est avantageusement délimitée par une marque colorée 6 visible sur la face externe de la collerette. Selon la disposition des orifices, cette marque sera plus ou moins en forme de cercle ou ovalisée.

Elle sera en forme de cercle lorsque les trois orifices seront groupés dans une zone triangulaire au centre de la collerette. Cette marque colorée en forme de cercle pourra passer par les trois angles d'un triangle ou légèrement plus à l'extérieur. Elle sera de forme ovalisée si les orifices sont alignés entre eux. Quelle que soit la disposition des orifices, la marque colorée 6 entourera la zone des orifices Z1 en laissant subsister un espace annulaire entre elle et le bord desdits orifices. Cette marque colorée constitue la limite entre la zone des orifices Z1 et la zone de solidarisation périphérique Z2 destinée à être solidarisée à l'aorte.

Cette marque ou ligne colorée 6 sert de repère pour la mise en place des points de suture qui suivront cette ligne, lors de la fixation.

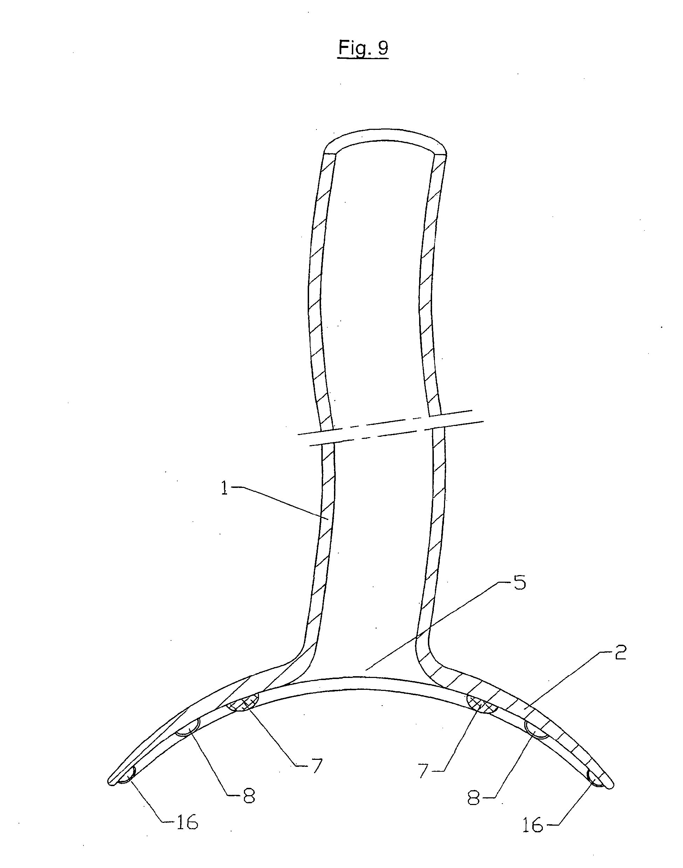

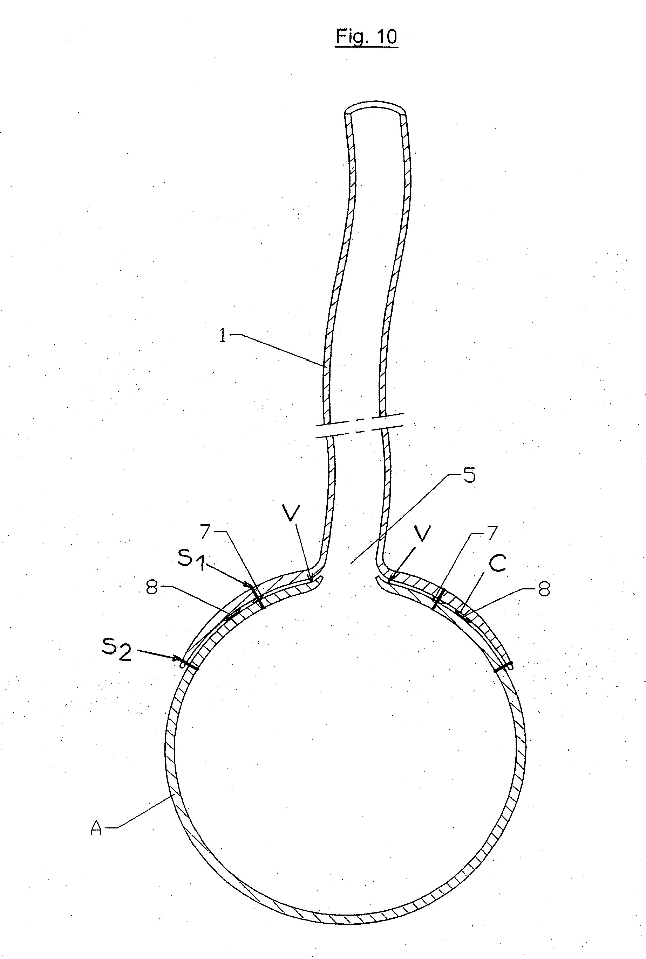

La face interne 4 est munie, en correspondance de la marque ou ligne colorée 6 visible sur la face externe, d'un épaississement ou renflement annulaire 7, appelé à remplir la fonction d'un joint d'étanchéité. Le joint d'étanchéité 7 décrit sur la face interne 4, le même trajet que la marque colorée 6 visible sur la face externe ou face supérieure 3, de sorte que la première suture qui sera effectuée en suivant le trajet de la marque colorée externe 6 va appliquer ce joint 7 sur la paroi artérielle et rendre étanche cette suture lorsque la circulation sera établie dans les conduits prothétiques 1a, 1b, 1c. De plus, ainsi réalisée, la suture se situe à distance des orifices qui seront ensuite créés dans un temps opératoire ultérieur bien distinct sur la paroi de l'aorte. Cette suture à distance des orifices (absence de suture bord à bord) va créer ultérieurement une sorte de valve naturelle V (figure 10) à partir de la paroi de l'aorte et qui va s'appliquer sur la paroi de la collerette sous l'effet de la pression artérielle. Cette disposition particulière obtenue après la mise en circulation du dispositif prothétique, isole la zone de suture et contribue à augmenter l'étanchéité procurée par le joint (figure 9).

Le joint 7 peut être constitué par une bandelette de Téflon ®, de Dacron ®, de PTFE, ou autre matériau souple biocompatible. Il peut être avantageusement réalisé en matériau tel que silicone ou polyuréthanne, qui peut facilement être solidarisé sur la face interne 4 de la collerette 2 et qui présente les caractéristiques de souplesse nécessaires pour remplir la fonction ou le rôle de joint.

L'emplacement recommandé des points de suture le long de la ligne colorée 6 peut être signalé par des petits traits transversaux 6a disposés régulièrement sur le trajet de ladite ligne colorée (figure 1).

Dans la zone de solidarisation Z2 et sous la face inférieure 4 de la collerette 2, est ménagée une loge ou canal annulaire 8 disposée autour de la zone des orifices 5a, 5b, 5c, ... Cette loge 8 est délimitée par une bande annulaire 8a de tissu biologique avantageusement bio résorbable. Cette bande de tissu est munie de petits trous et solidarisée, par ses bords latéraux 8a', 8a", à la face inférieure 4 de la collerette 2 qui constitue la paroi supérieure de ladite loge. Cette dernière située en sous-face de la collerette 2 est destinée à permettre l'injection d'une colle biologique et/ou d'un produit hémostatique lors de l'opération de fixation, de sorte que ladite colle ou ledit hémostatique traverse la paroi perméable 8a de la loge 8 et s'applique sur la paroi de l'aorte en constituant une zone de fixation alternative ou supplémentaire et une barrière imperméable autour de la zone des orifices et, éventuellement, autour du joint 7.

De manière préférée, au moins un cathéter souple 9a est disposé dans la loge annulaire 8 pour faciliter l'injection de la colle biologique et/ou du produit hémostatique, l'extrémité proximale 9a' de ce cathéter souple munie d'un organe de raccordement 9a" à un réservoir de colle et/ou du produit hémostatique (non représenté) traversant un pertuis 10 ménagé dans la collerette 2 et étant accessible à l'extérieur de celle-ci, de sorte à permettre son raccordement audit réservoir, puis son retrait lors de l'injection de la colle et/ou du produit hémostatique.

Lorsque la colle est constituée par une substance nécessitant son association à un catalyseur, un deuxième cathéter 9b est disposé dans la loge annulaire 8, avec une disposition inverse de celle du premier cathéter 9a. Ce deuxième cathéter 9b dont l'extrémité proximale 9b' est munie d'un organe de raccordement 9b" traverse également un deuxième pertuis 10 ménagé dans la collerette 2. Il est destiné à permettre l'injection du produit catalyseur dans la loge 8. Lors de l'opération d'injection de la colle et du produit catalysant, les cathéters 9a et 9b sont retirés successivement, en suivant des trajets inverses (dextrorsum et senestrorsum).

Le cathéter 9a et le cathéter 9b permettent l'injection de la colle et du produit catalysant au fur et à mesure de leur retrait de la loge 8, par une action de traction.

Lorsque la loge 8 a été remplie de la colle, éventuellement catalysée, celle-ci diffuse jusqu'à la paroi aortique à travers les petits orifices de la paroi inférieure perméable 8a de la loge 8. Cette diffusion de colle à travers les petits orifices de la paroi inférieure 8a de la loge 8, réalise une fixation efficace et imperméable de la collerette 2 sur la paroi de l'aorte, laquelle se trouve complétée par l'action du joint 7.

Selon un mode d'exécution possible, la loge 8 peut être délimitée par deux joints souples concentriques espacés 15a, 15b réalisés en matériau biocompatible, par exemple en silicone ou en polyuréthanne, rapportés sur la face interne 4 de la collerette.

Dans un tel cas, l'injection de la colle et/ou du produit hémostatique est réalisée après fixation de la collerette par suture de la collerette sur la paroi artérielle. L'injection se fait par au moins un pertuis ou orifice 10 débouchant dans la loge 8 et ménagé, à cet effet, dans la collerette.

Un joint annulaire supplémentaire 16 analogue aux joints 7, 15a, 15b, peut être disposé à l'extérieur de la loge annulaire 8, sur la face interne 4 de la collerette 2, par exemple à proximité de la bordure de ladite collerette. Ce joint annulaire supplémentaire correspond à une deuxième marque colorée visible sur la face externe de la collerette de manière identique à celle décrite pour le premier joint souple 7 et présentant les mêmes caractéristiques, pour faciliter la suture.

Tous ces éléments multiples de fixation ou d'étanchéité, situés dans la zone Z2 de solidarisation périphérique autour de la zone Z1, vont être plus ou moins combinés entre eux, permettant de fixer la collerette sur l'aorte sur une grande surface, augmentant considérablement la résistance de la fixation par rapport à une suture classique bord à bord.

On a représenté, à titre d'exemple, un dispositif prothétique comprenant trois branches collatérales ou conduits prothétique 1 a, 1b, 1c. On souligne encore toutefois que le dispositif prothétique selon l'invention peut comporter une seule branche collatérale, ou deux branches, voire plus de trois branches.

Les branches 1 a, 1b, 1c, peuvent avantageusement être réalisées en tissu prothétique identique à celui dans lequel est exécutée la collerette 2, ou compatible avec une fabrication en continuité avec celle-ci.

Lorsque les branches prothétiques collatérales 1a, 1 b, 1 c sont fabriquées en Dacron ®, elles seront avantageusement "crimpées", comme il est habituel de le faire, dans le domaine des conduits prothétiques, pour les rendre souples, variables en longueur et pour éviter les plicatures. Ces branches seront suffisamment longues pour leur permettre d'atteindre les vaisseaux artériels naissant de l'arche aortique, ou de toute autre portion d'aorte, depuis le lieu d'implantation du dispositif.

Les branches prothétiques collatérales 1 a, 1 b, 1 c, peuvent être de diamètre identique entre elles ou adaptées spécifiquement au diamètre de chaque artère à revasculariser.

Elles présentent une partie proximale 11 solidaire de la face externe 3 de la collerette et une partie distale 12 destinée à être anastomosée avec les vaisseaux à revasculariser par une technique chirurgicale classique.

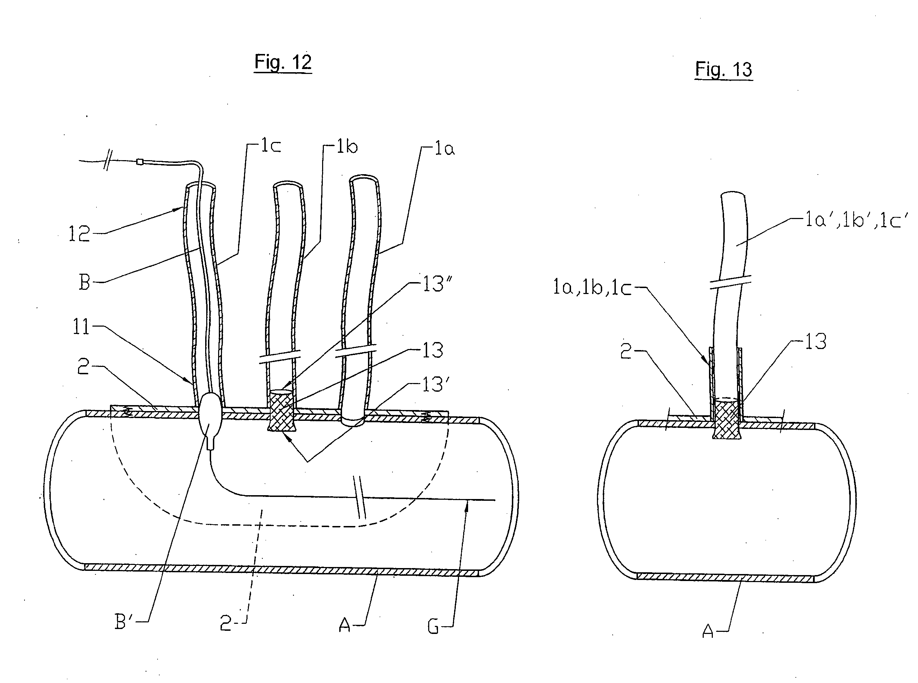

Selon un autre mode de réalisation possible, les tubes ou conduits prothétiques 1a, 1b, 1c, sont volontairement courts (par exemple de l'ordre de quatre à cinq centimètres). Ils seront prolongés ultérieurement par des segments de tubes 1 a', 1b', 1c' (figure 13) de calibre adapté pour rejoindre l'artère à revasculariser A1, ou A2 ou A3.

Les deux segments de tubes 1a-1a', 1b-1b', 1c-1c', pourront être réunis entre eux par une suture ou par l'utilisation d'un stent solidaire de l'extrémité proximale du tube 1 a', 1 b', 1 c', utilisé pour prolonger les tubes prothétiques courts. Sur la figure 13, un segment de tube de prolongement (1a', 1b', 1c') est représenté en position de raccordement au moyen d'un stent (13) à un conduit prothétique court (1 a, 1 b, 1 c) issu d'une collerette 2.

Les branches prothétiques peuvent être solidaires de la face externe ou face supérieure 3 de la collerette 2, par un tissage ou tricotage continu ou par simple suture.

On décrit ci-après un mode opératoire utilisant le dispositif prothétique de l'invention, pour réaliser un pontage vasculaire, à partir de l'aorte, sans interruption de la circulation sanguine dans cette dernière.

Contrairement à une prothèse vasculaire classique qui se coud sur les bords d'un orifice aortique réalisé préalablement, la collerette du dispositif de l'invention se fixe sur la paroi d'une portion intacte et circulante de l'aorte. Au moment de la fixation, il n'y a aucune communication entre l'aorte et l'extérieur comme c'est le cas avec l'utilisation d'une prothèse classique.

Cette particularité permet la suture sans avoir besoin d'aucune interruption de la circulation partiellement ou totalement au niveau de l'aorte. Ainsi, pendant la suture, le sang continue de circuler normalement vers l'aval. Ceci évite la souffrance des organes privés de sang situés en aval et le recours à une quelconque technique d'assistance circulatoire.

Dans un mode de réalisation avantageux, la collerette 2 peut être fixée sur l'aorte par deux surjets, l'un (S1) qui va suivre la marque colorée 6 et va appliquer le joint 7 sur la paroi de l'aorte A, l'autre (S2) prenant appui sur le bord périphérique de la collerette 2, siège éventuellement d'un deuxième joint 16 et suivant la deuxième marque colorée correspondante. Entre les deux lignes de sutures S1, S2, de la colle biologique C et/ou un produit hémostatique injecté(e) dans la loge 8 prévue à cet effet, par les deux cathéters 9a, 9b, vient compléter la fixation de la collerette 2 sur l'aorte A (figure 10). À ce stade, il n'y a pas de communication entre l'aorte A et les conduits prothétiques 1 a, 1b, 1c. Le dispositif prothétique n'est pas circulant.

La mise en communication de l'aorte A avec les conduits prothétiques 1a, 1b, 1c, au niveau des orifices de la collerette 5a, 5b, 5c, est réalisée une fois que la collerette 2 est parfaitement fixée sur la paroi de l'aorte A. Elle est exécutée avec les principes des techniques endovasculaires qui ne nécessitent aucun clampage. A titre d'exemple, on cite l'une des techniques pouvant être utilisée pour effectuer cette mise en communication.
A/ Avec une aiguille introduite par l'une des branches collatérales prothétiques 1 a, 1b, 1 c, on pique la paroi de l'aorte au centre de l'orifice correspondant de cette branche et un guide de radiologie G (figure 12) est introduit dans la lumière aortique pratiquée.
B/ Ensuite, grâce au guide G, on introduit un cathéter B à ballonnet B' sur ledit guide et un orifice est créé dans la paroi aortique en regard de l'orifice de la branche prothétique considérée, en gonflant le ballonnet B qui dilate la paroi. On peut également réaliser cet orifice en introduisant un cathéter de gros calibre ayant un nez profilé en forme de cône.
C/ Dans l'orifice ainsi créé, on déploie éventuellement un stent 13 couvert ou non, qui a pour rôle de régulariser l'orifice et de solidariser encore mieux l'aorte et la collerette avec sa branche. Ce stent 13 peut être couvert et avoir une forme particulière, par exemple, avantageusement, une forme de rivet ou d'entonnoir pour mieux se fixer et ouvrir l'orifice. La partie évasée 13' du stent étant du côté aortique, la partie plus effilée 13" se trouvant dans la branche prothétique collatérale. Ce stent 13 contribuera à isoler encore plus la ligne de suture interne S1 de la partie circulante.

Dans le mode de réalisation particulier cité précédemment où le ou les conduits prothétiques 1a, 1b, 1c, sont volontairement courts, ce stent peut être solidaire de l'extrémité proximale du tube utilisé pour prolonger le ou les conduits prothétiques jusqu'aux branches collatérales A1, ou A2 ou A3 à revasculariser.

Une fois l'ouverture pratiquée, une pince (ou clamp) maintiendra momentanément fermée cette branche prothétique jusqu'à la réalisation de l'anastomose distale sur la branche anatomique collatérale considérée A1, ou A2 ou A3.

La mise en communication de la lumière aortique avec les deux autres branches s'opérera de la même manière.

Après les étapes précédentes, le sang arrive dans les branches 1a, 1b, 1c, du dispositif prothétique. La partie distale de ces branches est ensuite successivement anastomosée avec les vaisseaux anatomiques A1, A2, A3, qui doivent être revascularisés, selon une technique chirurgicale classique.

On observe que le dispositif prothétique selon l'invention peut comporter un tube souple, supplémentaire (non représenté) raccordé à la face externe 3 de la collerette 2 et de matière identique à celle des conduits prothétiques 1a, 1b, 1c, dédié au passage de l'instrumentation nécessaire pour la mise en place d'une endoprothèse dans l'aorte lorsque celle-ci présente un anévrysme. Ce tube supplémentaire débouchant dans un orifice traversant ladite collerette 2, et disposé dans la zone des orifices Z1.

Dans le cas d'une indication de revascularisation pour occlusion des branches collatérales A1, A2, A3, le geste opératoire qui vient d'être décrit peut être suffisant.

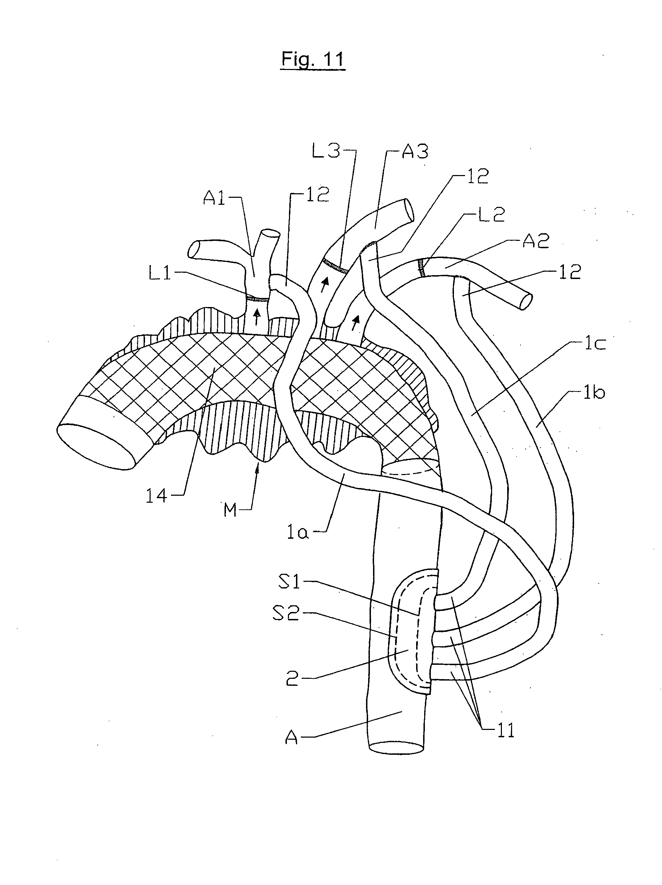

S'il s'agit d'une revascularisation des branches de l'arche aortique pour anévrysme (Figure 11), après la revascularisation des deux premières branches collatérales anatomiques, on peut exclure l'arche par une endoprothèse 14 en utilisant soit la troisième branche prothétique du dispositif non encore anastomosée, soit par le tube supplémentaire précédemment décrit et prévu à cet effet. Celle-ci ou celui-ci permettra d'introduire le cathéter contenant l'endoprothése.

On peut aussi finir de revasculariser normalement la troisième branche A3 et exclure l'arche aortique par une endoprothèse introduite par voie fémorale. L'exclusion peut être faite à n'importe quel moment vu que le sang arrive déjà au niveau des branches collatérales. L'origine des branches revascularisées A1, A2, A3, est en général ligaturée en amont de l'anastomose distale (ces ligatures L1, L2, L3 sont représentées schématiquement sur la figure 11).

La figure 11 montre le résultat final avec la collerette 2 du dispositif fixée sur l'aorte (A), les conduits prothétiques 1 a, 1 b, 1 c dudit dispositif dont les parties distales 12 sont anastomosées avec les collatérales A1, A2, A3, de l'aorte A et l'endoprothèse aortique 14 qui exclut et traite l'anévrysme, ou partie malade M.

On souligne encore que grâce à cette méthode, et au dispositif prothétique selon l'invention, il est ainsi possible de pratiquer des anastomoses vasculaires sur l'aorte ou tout autre vaisseau sans aucun clampage partiel ou total. Au niveau de l'arche aortique, cela évite une circulation extracorporelle avec hypothermie et arrêt circulatoire.

Cette caractéristique permet une geste opératoire très simple dont le but est de réduire la lourdeur des interventions et la mortalité des patients. Il n'est plus nécessaire d'effectuer un clampage de l'aorte, et cela est plus particulièrement avantageux pour l'aorte thoracique et thoraco-abdominale. Cette simplification de la technique de fixation du dispositif prothétique permet également de réduire la taille des incisions thoraciques et/ou abdominales pour accéder à l'aorte, voire d'envisager de la réaliser avec un abord vidéoscopique de l'aorte, ce qui aura pour effet d'éliminer complètement les incisions.

## Revendications

1. Dispositif prothétique, pour réaliser un pontage vasculaire à partir d'un vaisseau artériel, en particulier à partir de l'aorte, pouvant présenter des branches collatérales importantes, **caractérisé en ce qu'**il comprend un ou plusieurs tubes prothétiques (1 a, 1b, 1c) dont l'une des extrémités (11) est solidaire d'une collerette de fixation souple (2) comportant une face externe (3) et une face interne (4) destinée à être appliquée sur la paroi externe dudit vaisseau artériel, **caractérisé en ce que** cette collerette étant pourvue d'un ou plusieurs orifices (5a, 5b, 5c) disposés dans une zone centrale (Z1) dite zone des orifices lesquels communiquent avec le ou lesdits tubes prothétiques (1a, 1b, 1c), cette collerette comportant une zone de solidarisation (Z2) disposée autour de ladite zone des orifices (Z1), à distance du bord dudit ou desdits orifices et étant destinée à être fixée sur la paroi externe d'un vaisseau artériel, la face interne de cette zone de solidarisation étant pourvue d'au moins un moyen (7, 8, 15a-15b, 16) permettant de créer une zone annulaire d'étanchéité autour de ladite zone des orifices.

2. Dispositif prothétique selon la revendication 1, **caractérisé en ce que** la face interne (4) de la zone de solidarisation (Z2) comporte au moins un joint annulaire souple (7) disposé autour de la zone des orifices (Z1).

3. Dispositif prothétique suivant la revendication 1, **caractérisé en ce que** la face interne de la zone de solidarisation (Z2) comporte un espace ou loge annulaire (8) disposée autour de la zone des orifices (Z1) et permettant l'injection d'un produit, par exemple d'une colle ou d'un hémostatique avantageusement bio résorbable, destiné à constituer une zone de fixation annulaire formant joint étanche.

4. Dispositif prothétique selon la revendication 3, **caractérisé en ce que** la loge annulaire (8) est délimitée par une bande annulaire inférieure perméable (8a) destinée à être appliquée sur la paroi externe d'un vaisseau sanguin, en particulier sur la paroi aortique, cette bande annulaire étant pourvue de petits trous permettant la diffusion, en sous face de ladite collerette, d'une colle biologique et/ou d'un produit hémostatique avantageusement bio résorbable injecté(e) dans ladite loge.

5. Dispositif prothétique selon la revendication 4, **caractérisé en ce que** la loge annulaire (8) est constituée par une bande annulaire de tissu biologique (8a) avantageusement bio- résorbable munie de petits trous et solidarisée par ses bords latéraux (8a', 8a") à la face inférieure (3) de la collerette (2).

6. Dispositif prothétique selon les revendications 2 et 3, ou 4 ou 5, **caractérisé en ce que** la loge annulaire (8) est disposée à l'extérieur du joint souple (7).

7. Dispositif prothétique selon la revendication 3, **caractérisé en ce que** la loge annulaire (8) est délimitée, latéralement par deux joints concentriques espacés (15a, 15b) disposés sur la face interne de la collerette (2) qui comporte au moins un pertuis (10) débouchant dans ladite loge.

8. Dispositif prothétique selon l'une quelconque des revendications 3 à 6, **caractérisé en ce qu'**un joint annulaire supplémentaire (16) est disposé à l'extérieur de la loge annulaire (8) sur la face interne de la collerette (2).

9. Dispositif prothétique suivant l'une quelconque des revendications 2, 6, 7 ou 8, **caractérisé en ce que** les joints annulaires (7, 15a, 15b, 16) sont constitués par un bourrelet de matériau souple biocompatible, par exemple de silicone ou de polyuréthanne.

10. Dispositif prothétique suivant l'une quelconque des revendications 3, 4, 5 ou 6, **caractérisé en ce qu'**au moins un cathéter souple (9a) est disposé dans la loge annulaire (8) pour l'injection d'une colle biologique et/ou d'un produit hémostatique, l'extrémité proximale (9a') de ce cathéter traversant un pertuis (10) ménagé dans la collerette (2), et étant accessible sur la face externe (3) de cette dernière, de sorte à permettre son raccordement à un réservoir de colle biologique ou de produit hémostatique préférentiellement bio résorbable et son retrait, lors de l'injection de ladite colle et/ou dudit produit hémostatique.

11. Dispositif prothétique selon la revendication 10, **caractérisé en ce que** deux cathéters souples (9a, 9b) sont disposés, avec une disposition inverse, dans la loge annulaire (8) ménagée en sous-face de la collerette (2) de fixation, l'un pour l'injection d'une colle biologique, l'autre pour l'injection d'un produit catalysant, ces cathéters pouvant être retirés successivement en suivant des trajets inverses (dextrorsum et senestrorsum).

12. Dispositif prothétique suivant l'une quelconque des revendications 2 ou 9, **caractérisé en ce que** la zone des orifices (Z1) est délimitée par une marque annulaire colorée (6) visible sur la face externe (3) de la collerette (2) et disposée en correspondance du joint annulaire (7) et/ou **en ce que** le joint annulaire (16) situé sur la face interne de la collerette (2) à proximité de la périphérie de cette dernière correspond à une marque annulaire colorée située sur la face externe (3).

13. Dispositif prothétique selon l'une quelconque des revendications 1 à 12, **caractérisé en ce que** la collerette souple (2) présente la forme d'une portion de cylindre droit lorsque le dispositif est destiné à être implanté sur une portion d'aorte rectiligne, ou la forme d'une portion de cylindre coudé lorsque ledit dispositif doit être implanté sur une portion courbe de l'aorte.

14. Dispositif prothétique selon l'une quelconque des revendications 1 à 13, **caractérisé en ce qu'**il comporte un tube supplémentaire raccordé à la collerette (2) et débouchant dans un orifice traversant cette dernière, ce tube supplémentaire étant dédié au passage de l'instrumentation opératoire.

15. Dispositif prothétique selon l'une quelconque des revendications 1 à 14, **caractérisé en ce qu'**il comprend des stents (13) aptes à être installés au niveau de l'origine des tubes prothétiques (1a, 1b, 1c) après ouverture de la communication avec l'aorte ou tout autre vaisseau sur lequel ledit dispositif prothétique serait implanté.

16. Dispositif prothétique selon l'une quelconque des revendications 1 à 15, **caractérisé en ce que** le ou les conduits prothétiques (1a, 1b, 1c) sont volontairement courts et des conduits prothétiques de calibre adapté (1a', 1b', 1c'), prolongent les conduits courts (1a, 1b, 1c) issus de la collerette, pour atteindre les branches à revasculariser (A1, A2, A3), et **en ce que** les conduits prothétiques (1 a', 1b', 1c') utilisés pour prolonger les tubes prothétiques (1a, 1b, 1c) issus de la collerette (2), sont solidaires d'un stent pouvant être installé au niveau de l'origine des tubes prothétiques courts (1a, 1b, 1c).

## Patentansprüche

1. Prothese zur Herstellung eines Gefäßbypasses aus einem arteriellen Blutgefäß, insbesondere aus der Aorta, die beträchtliche Seitenäste umfassen kann, **dadurch gekennzeichnet, dass** sie einen oder mehrere Prothesenschläuche (1a, 1b, 1c) umfasst, deren eines Ende (11) an einer flexiblen Befestigungskrause (2) befestigt ist, welche eine Außenfläche (3) und eine Innenfläche (4) umfasst, die zum Anbringen an die Außenwand des arteriellen Blutgefäßes vorgesehen ist, **dadurch gekennzeichnet, dass** die mit einer oder mehreren Öffnungen (5a, 5b, 5c) bereitgestellte Krause, welche in einem zentralen Bereich (Z1) namens Bereich der Öffnungen angeordnet sind, die mit dem oder den Prothesenschläuchen (1a, 1b, 1c) verbunden sind, wobei die Krause einen Befestigungsbereich (Z2) umfasst, der vom Rand der Öffnung (en) beabstandet um den Bereich der Öffnungen (Z1) herum angeordnet und zum Befestigen an die Außenwand eines arteriellen Blutgefäßes vorgesehen ist, wobei die Innenfläche des Befestigungsbereichs mit mindestens einem Mittel (7, 8, 15a-15b, 16) bereitgestellt wird, welches das Ausbilden eines ringförmigen Versiegelungsbereichs um den Öffnungsbereich herum gestattet.

2. Prothese nach Anspruch 1, **dadurch gekennzeichnet, dass** die Innenfläche (4) des Befestigungsbereichs (Z2) mindestens eine ringförmige flexible um den Öffnungsbereich (Z1) herum angeordnete Dichtung (7) umfasst.

3. Prothese nach Anspruch 1, **dadurch gekennzeichnet, dass** die Innenfläche des Befestigungsbereichs (Z2) einen ringförmigen Raum oder eine ringförmige um den Öffnungsbereich (Z1) herum angeordnet Zelle (8) umfasst, und die Injizierung eines Produkts gestattet, wie beispielsweise die eines Haftmittels oder eines vorzugsweise biologisch resorbierbaren blutstillenden Mittels, welches dafür bestimmt ist, einen ringförmigen Befestigungsbereich zu bilden, der eine feuchtigkeitssichere Dichtung ausbildet.

4. Prothese nach Anspruch 3, **dadurch gekennzeichnet, dass** die ringförmige Zelle (8) durch ein unteres durchlässiges ringförmiges Band (8a) begrenzt wird, das zum Anbringen an die Außenwand eines Blutgefäßes, insbesondere an die Aortenwand vorgesehen ist, wobei das ringförmige Band mit kleinen Löchern bereitgestellt ist, welche die Diffusion, auf der Unterseite der Krause, eines biologischen Haftmittels und/oder eines vorzugsweise biologisch resorbierbaren blutstillenden Produkts, das in die Zelle injiziert wird, gestatten.

5. Prothese nach Anspruch 4, **dadurch gekennzeichnet, dass** die ringförmige Zelle (8) aus einem ringförmigen Band aus biologischem vorzugsweise biologisch resorbierbarem Gewebe (8a) besteht, das kleine Löcher umfasst und über seine Seitenränder (8a', 8a'') an der Innenfläche (3) der Krause (2) befestigt ist.

6. Prothese nach den Ansprüchen 2 und 3, oder 4 oder 5, **dadurch gekennzeichnet, dass** die ringförmige Zelle (8) außen an der flexiblen Dichtung (7) angeordnet ist.

7. Prothese nach Anspruch 3, **dadurch gekennzeichnet, dass** die ringförmige seitlich durch zwei beabstandete konzentrische Dichtungen (15a, 15b) begrenzte Zelle (8) an der Innenfläche der Krause (2) angeordnet ist, welche mindestens einen in die Zelle führenden Kanal (10) umfasst.

8. Prothese nach einem der Ansprüche 3 bis 6, **dadurch gekennzeichnet, dass** eine zusätzliche ringförmige Dichtung (16) außen an der ringförmigen Zelle (8) an der Innenfläche der Krause (2) angeordnet ist.

9. Prothese nach einem der Ansprüche 2, 6, 7 oder 8, **dadurch gekennzeichnet, dass** die ringförmigen Dichtungen (7, 15a, 15b, 16) aus einem Dichtungsband aus flexiblem biologisch kompatiblem Material bestehen, beispielsweise aus Silikon oder Polyurethan.

10. Prothese nach einem der Ansprüche 3, 4, 5 oder 6, **dadurch gekennzeichnet, dass** mindestens ein flexibler Katheter (9a) zum Injizieren eines biologischen Haftmittels und/oder eines blutstillenden Produkts in der ringförmigen Zelle (8) angeordnet ist, wobei das nahe Ende (9a') des Katheters einen Kanal (10) durchquert, welcher in der Krause (2) angeordnet ist, und an deren Außenfläche (3) zugänglich ist, um dessen Verbindung mit einem Reservoir mit biologischem Haftmittel oder vorzugsweise biologisch resorbierbarem blutstillendem Produkt und bei der Injizierung des Haftmittels und/oder des blutstillenden Produkts dessen Entfernung zu gestatten.

11. Prothese nach Anspruch 10, **dadurch gekennzeichnet, dass** zwei flexible Katheter (9a, 9b), mit umgekehrter Anordnung, in der ringförmigen Dichtung (8), die an der Unterseite der Befestigungskrause (2), angeordnet sind, und zwar eine für die Injizierung eines biologischen Haftmittels und der andere für die Injizierung eines katalysierenden Produkts, und wobei die Katheter aufeinanderfolgend entgegengesetzten Richtungen folgend (nach rechts und nach links) herausgezogen werden können.

12. Prothese nach einem der Ansprüche 2 oder 9, **dadurch gekennzeichnet, dass** der Öffnungsbereich (Z1) durch eine farbige Ringmarkierung (6) begrenzt ist, die an der Außenfläche (3) der Krause (2) sichtbar und der ringförmigen Dichtung (7) entsprechend angeordnet ist und/oder, dass die an der Innenfläche der Krause (2) in Nähe deren Peripherie angeordnete ringförmige Dichtung (16) einer farbigen Ringmarkierung entspricht, die sich an der Außenfläche (3) befindet.

13. Prothese nach einem der Ansprüche 1 bis 12, **dadurch gekennzeichnet, dass** die flexible Krause (2) die Form eines geraden Zylinderabschnitts umfasst, wenn die Prothese zum Implantieren an einen geradlinigen Aortenabschnitt vorgesehen ist, oder die Form eines gebogenen Zylinderabschnitts aufweist, wenn die Prothese auf einen gebogenen Abschnitt der Aorta zu implantieren ist.

14. Prothese nach einem der Ansprüche 1 bis 13, **dadurch gekennzeichnet, dass** sie einen zusätzlichen Schlauch umfasst, der mit der Krause (2) verknüpft ist und in einer Öffnung mündet, welche letztere durchquert, wobei der zusätzliche Schlauch zum Durchführen von chirurgischen Instrumenten vorgesehen ist.

15. Prothese nach einem der Ansprüche 1 bis 14, **dadurch gekennzeichnet, dass** sie Stents (13) umfasst, die auf der Höhe des Anfangs der Prothesenschläuche (1a, 1b, 1c) nach dem Öffnen der Verbindung mit der Aorta oder jedem anderen Gefäß, an dem die Prothese implantiert wird, anbringbar sind.

16. Prothese nach einem der Ansprüche 1 bis 15, **dadurch gekennzeichnet, dass** der oder die Prothesenschläuche (1a, 1b, 1c) absichtlich kurz sind, und dass die Prothesenleitungen mit angepasstem Kaliber (1a', 1b', 1c') die kurzen Leitungen (1a, 1b, 1c), die aus der Krause führen, verlängern, um die zu revaskularisierenden Zweige (A1, A2, A3) zu erreichen, und dass die Prothesenleitungen (1a', 1b', 1c'), die verwendet werden, um die Prothesenschläuche (1a, 1b, 1c), die aus der Krause (2) führen, fest mit einem Stent verbunden sind, der auf der Höhe des Anfangs der kurzen Prothesenleitungen (1a, 1b, 1c) installierbar ist.

## Claims

1. **Prosthetic device for performing a vascular bypass on an arterial vessel, particularly on the aorta, which may have important collateral branches, characterised in that it comprises one or more prosthetic tubes (1a, 1b, 1c) one of whose ends (11) is connected with a flexible fixing flange (2) comprising an outer face (3) and an inner face (4) intended to be applied to the outer wall of the said arterial vessel, characterised in that the said flange is provided with one or more apertures (5a, 5b, 5c) arranged in a central area (Z1) and the said apertures communicate with the said prosthetic tubes (1a, 1b, 1c), the said flange comprising a connection area (Z2) arranged around the said area of apertures (Z1), at a distance from the edge of the said aperture or apertures and intended to be fixed to the outer wall of an arterial vessel, the inner face of the said connection area being provided with at least one means (7, 8, 15a-15b, 16) allowing the creation of an annular sealing area around the said area of apertures.**

2. **Prosthetic device according to claim according to claim 1, characterised in that the inner face (4) of the connection area (Z2) comprises at least one flexible annular seal (7) arranged around the area of apertures (Z1).**

3. **Prosthetic device according to claim 1, characterised in that the inner face of the connection area (Z2) comprises an annular space or cavity (8) arranged around the area of apertures (Z1) and allowing the injection of a product, for example a glue or a haemostatic product, advantageously bioresorbable, intended to serve as an annular fixing area forming a leak-free seal.**

4. **Prosthetic device according to claim 3, characterised in that the annular cavity (8) is delimited by a permeable lower annular band (8a) intended to be applied to the outer wall of a blood vessel, particularly the wall of the aorta, the said annular band being provided with small holes allowing the diffusion, on the underside of the said flange, of a biological glue and/or a haemostatic product, advantageously bioresorbable, injected into the said cavity.**

5. **Prosthetic device according to claim 4, characterised in that the annular cavity (8) is constituted by an annular band of biological tissue (8a), advantageously bioresorbable, provided with small holes and connected by its lateral edges (8a', 8a") to the lower face (3) of the flange (2).**

6. **Prosthetic device according to claims 2 and 3, or 4 or 5, characterised in that the annular cavity (8) is arranged outside the flexible seal (7).**

7. **Prosthetic device according to claim 3, characterised in that the annular cavity (8) is delimited laterally by two spaced concentric seals (15a, 15b) arranged on the inner face of the flange (2), which comprises at least one narrow passage (10) emerging in the said cavity.**

8. **Prosthetic device according to any one of claims 3 to 6, characterised in that an additional annular seal (16) is arranged outside the annular cavity (8) on the inner face of the flange (2).**

9. **Prosthetic device according to any one of claims 2, 6, 7 or 8, characterised in that the annular seals (7, 15a, 15b, 16) are formed by a bead of biocompatible flexible material, for example silicone or polyurethane.**

10. **Prosthetic device according to any one of claims 3, 4, 5 or 6, characterised in that at least one flexible catheter (9a) is arranged in the annular cavity (8) for the injection of a biological glue and/or a haemostatic, the proximal extremity (9a') of the said catheter passing through a narrow passage (10) provided in the flange (2) and being accessible on the outer face (3) of the same, so as to allow its connection to a reservoir of biological glue or haemostatic product, preferably bioresorbable, and its withdrawal, during the injection of the said glue and/or haemostatic product.**

11. **Prosthetic device according to claim 10, characterised in that two flexible catheters (9a, 9b) are provided, with an inverse arrangement, in the annular cavity (8) arranged on the underside of the fixing flange (2), one for the injection of a biological glue and the other for the injection of a catalyst, the said catheters being capable of being successively removed in inverse directions (to the right and to the left).**

12. **Prosthetic device according to any one of claims 2 or 9, characterised in that the area of apertures (Z1) is delimited by a coloured annular marking (6) visible on the outer face (3) of the flange (2) and arranged coincidentally with the annular seal (7) and/or in such a way that the annular seal (16) situated on the inner face of the flange (2) close to the periphery of the same coincides with a coloured annular marking situated on the outer face (3).**

13. **Prosthetic device according to any one of claims 1 to 12, characterised in that the flexible flange (2) has the form of a straight cylinder portion when the device is intended to be implanted on a straight portion of aorta, or the form of an angled cylinder portion when the said device is to be implanted on a curved portion of aorta.**

14. **Prosthetic device according to any one of claims 1 to 13, characterised in that it comprises an additional tube connected to the flange (2) and emerging in an aperture passing through the same, the said additional tube being dedicated to the passage of the operating instruments.**

15. **Prosthetic device according to any one of claims 1 to 14, characterised in that it comprises stents (13) suitable for installation at the beginning of the prosthetic tubes (1a, 1b, 1c) after the opening of communication with the aorta or any other vessel on which the said prosthetic device is to be implanted.**

16. **Prosthetic device according to any one of claims 1 to 15, characterised in that the prosthetic tubes (1a, 1b, 1c) are intentionally short and prosthetic tubes of a suitable calibre (1a' 1b', 1c') extend the short tubes (1a, 1b, 1c) emerging from the flange in order to reach the branches to be revascularised (A1, A2, A3), and in that the prosthetic tubes (1a', 1b', 1c') used for extending the prosthetic tubes (1a, 1b, 1c) emerging from the flange (2) are connected to a stent capable of being installed at the beginning of the short prosthetic tubes (1a, 1b, 1c).**
